# EUROPEAN PATENT APPLICATION

(11) **EP 4 085 913 A1**
(43) Date of publication of application: **09.11.2022**
(21) Application number: 22171910.7
(22) Date of filing: 05.05.2022
(51) Int. Cl.: A61K 31/455, A61K 33/30, A61K 33/34, A61K 38/06, A61K 31/19, A61K 31/355, A61K 31/375, A61K 31/728, A61K 38/56, A61P 17/10, A61K 9/00, A61Q 19/00

(54) **FORMULATION COMPRISING NICOTINAMIDE, ZINC, COPPER AND GLUTATHIONE FOR TREATING ACNE ROSACEA**

(30) Priority: 07.05.2021 IT 202100011750
(71) Applicant: Iromed Group S.r.l., 00144 Roma (RM) (IT)
(72) Inventor: CAMPIONE, Elena, Roma RM (IT); COSIO, Terenzio, Torino TO (IT)
(74) Representative: Di Giovine, Paolo

(57) **Abstract**

The present invention refers to a composition comprising nicotinamide, zinc, copper and reduced glutathione (GSH) to offer relief from the unpleasant sensations typical of hypersensitive skin, protecting the skin barrier and soothing irritation, helping to balance the secretion of sebum and improve cell metabolism, increasing skin permeability.

## Description

### FIELD OF THE INVENTION

The present invention refers to a composition comprising nicotinamide, zinc, copper and reduced glutathione (GSH), to offer relief from the unpleasant sensations typical of hypersensitive skin, protecting the skin barrier and soothing irritations, helping to balance the secretion of sebum and improve cell metabolism, increasing the permeability of the skin. The present invention furthermore refers to said compositions for use in the treatment of acne rosacea and its symptoms.

### STATE OF THE ART

Acne rosacea is a chronic inflammatory disease of the skin, mainly affecting adults with fair complexion and hair (hence it is known as the "Curse of the Celts"). The onset of the dermatological pathology is accompanied by redness mainly on the cheeks, nose, chin and forehead. Acne rosacea tends to have a progressive course, therefore the symptoms tend to worsen over time: the redness tends to increase until it becomes persistent. If not appropriately treated, acne rosacea can cause skin lesions, swelling of the nose and also affect the region around the eyes (ocular rosacea). For some patients, rosacea has a cyclical course: the symptoms can worsen for a period limited to a few weeks or months, and then diminish before worsening again. Acne rosacea is a primitively vascular disorder: the early signs are the typical redness, due to alterations of the venous flows; the first skin lesions are pathological dilations of the blood capillaries.

There is no real cure for acne rosacea, but treatment can alleviate the symptoms and control the progression thereof. A dermatologist (doctor specializing in skin diseases) can advise on the most appropriate medical treatment, in addition to recommending certain moisturizers, delicate cleansers, sun creams and other products to improve skin health.

There are various alternatives for treating rosacea: a combination of drugs may be necessary, applied at topical level (lotion, cream or gel) and for oral administration (pills, capsules or tablets). The duration of the treatment depends on the type and gravity of the symptoms. Long-term treatment is often necessary to prevent the symptoms re-occurring.

Since the most suitable drugs for completely curing the disease have not yet been identified, the substances listed below do not provide a cure for rosacea; rather, these treatments are directed at improving the person's appearance, in addition to reducing any inflammation associated with rosacea.

The acne rosacea drugs for local application perform an antibacterial and anti-inflammatory activity: they comprise antibiotics like metronidazole, tetracycline, erythromycin, sulfacetamide sodium-sulphur or azelaic acid, retinoids for topical use, like isotretinoin, or corticosteroids, like loteprednol; and can be included in therapeutic maintenance programmes, for control of the symptoms. These topical applications can also have side effects such as skin irritation, reddening, dry skin or smarting.

Nature also offers us some useful remedies for treating rosacea: herbalist shops stock some products purposely formulated to treat rosacea, for example artichoke, chicory, walnut, rosemary, Butcher's Broom and vine. These natural remedies act on the microcirculation, increasing the resistance of the capillaries and countering their fragility, a typical characteristic of rosacea.

In the state of the art, the problem of providing methods and compositions able to treat this pathology and alleviate its symptoms such as acne, reddening of the face (forehead, nose, cheeks), formation of telangiectasia on the face, red eyes, tendency to lachrymation and to reddening and tingling sensation, at times associated with itching, deriving from acne rosacea, is still a deeply felt problem.

### SUMMARY OF THE INVENTION

The technical problem posed and solved by the present invention is that of providing a natural anti-inflammatory composition for acne rosacea able to improve the microcirculation of the face, balance the secretion of sebum, improve cell metabolism, increase skin permeability and rapidly eliminate inflammation of the skin.

This problem is solved via the use of a composition comprising nicotinamide, zinc, copper and reduced glutathione (GSH) according to claim 1.

The association of the nicotinamide with the glutathione allows adequate glutathione levels to be maintained even during the inflammatory stress of acne rosacea.

The NADPH oxidase inhibitors like zinc therefore have a protective influence taking account of the reduction in the formation of ROS.

At intracellular level, copper participates, together with glutathione, in reducing the oxidative stress underlying acne rosacea. To achieve a lasting and adequate effect of the copper and glutathione at intracellular level, the glutathione must be provided with the nicotinamide, which ensures regeneration of the glutathione in reduced form. The zinc acts downstream, in turn favouring the formation of reduced nicotinamide, which will act as a substrate to form the reduced glutathione, which in turn acts in synergy with the copper to reduce the ROS and also to allow the latter to enter the cell.

Preferred characteristics of the present invention are the subject of the dependent claims.

The present invention also concerns a cosmetic product comprising or consisting of the composition according to any one of the embodiments described here in the form of serums, face masks, make-up remover wipes, cleansing wipes, moisturizing creams, lifting creams, moisturizing gels, make-up removers, exfoliants, toners, micellar water and/or make-up products.

Lastly, a further subject of the present invention is said composition for use in the treatment of acne rosacea, in particular for preventing, reducing and/or eliminating symptoms caused by acne rosacea and/or flushing and/or couperouge and/or capillary fragility and/or telangiectasia and/or facial pustulosis.

### DETAILED DESCRIPTION OF THE FIGURES

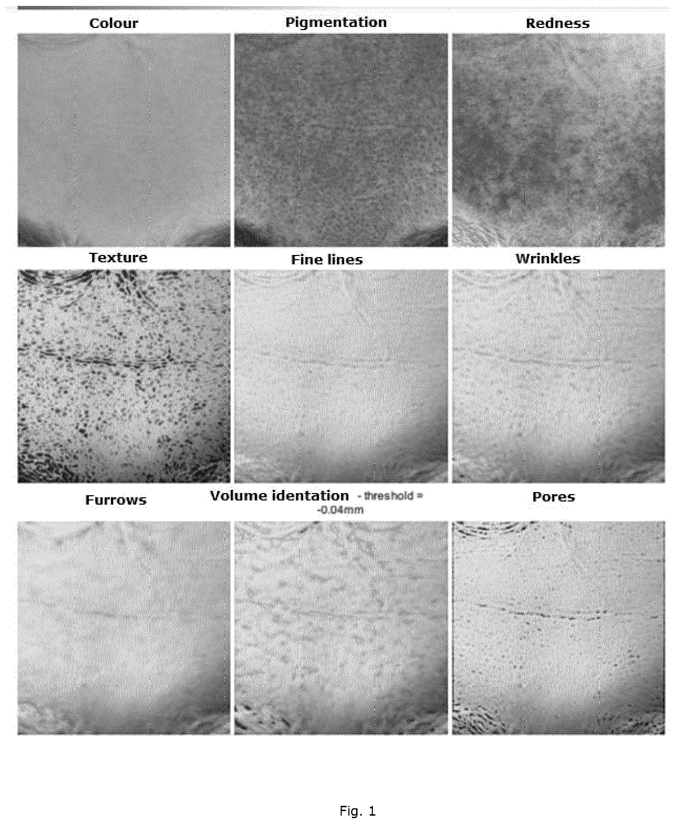

Figure 1 shows the 9 parameters examined during *in vivo* clinical evaluation on a human, at time T0, identified as baseline, namely before the beginning of the treatment. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

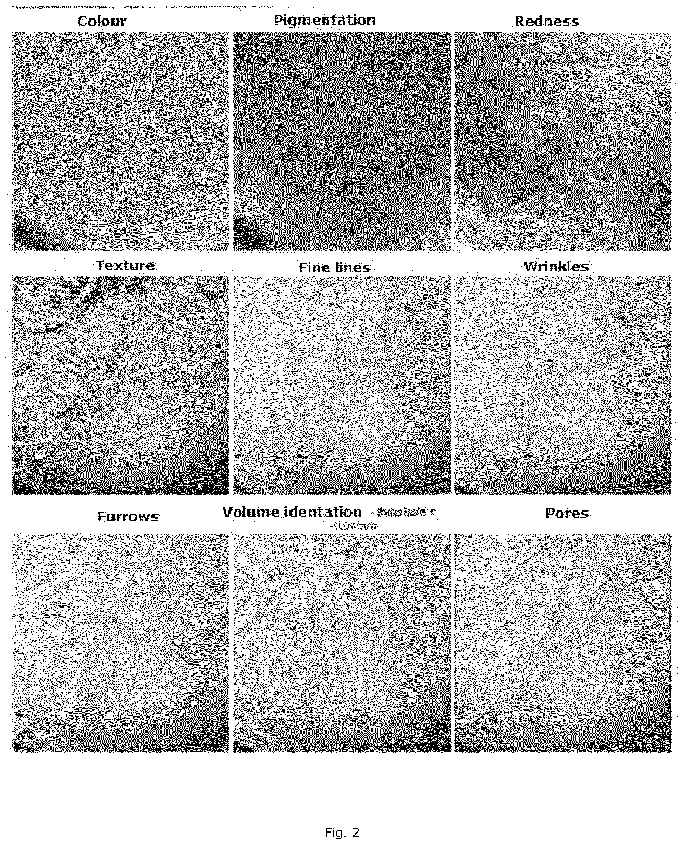

Figure 2 shows how after 21 days of application of the composition applied in Figure 1, all 9 parameters examined have a positive variation. The colour parameter is attenuated with respect to the basal parameter; the pigmentation parameter is reduced, underlining the topical effect of reduction of the melanin and the haemosiderin; the redness parameter is reduced following the vascular protective effect exercised by the vitamin K and resveratrol; the texture, fine lines, furrows and wrinkles parameters are reduced, underlining the synergic action of the composition comprising Hyaluronic Acid (0.1%); Nicotinamide (0.4%); Papain (0.1%); Copper (0.2%) Zinc (0.1%); Glutathione (0.1%); Vitamin C (0.1%); 18-beta Glycyrrhetic Acid (0.2%); Vitamin E (0.7%); with spf filters. Furthermore, a considerable improvement is also noted in the pores parameter in terms of quantity and quality.

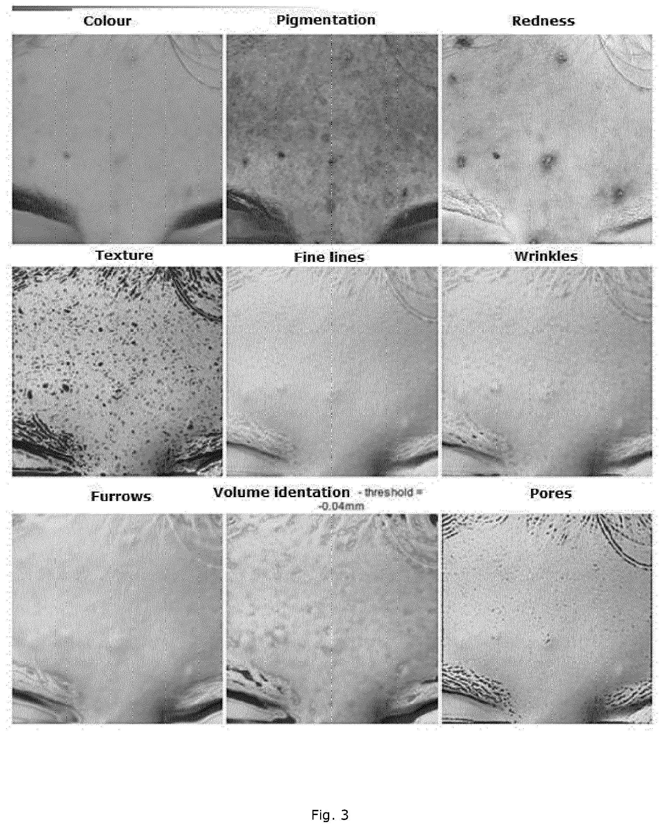

Figure 3 shows the 9 parameters examined during *in vivo* clinical evaluation on a human, at time T0, identified as baseline, namely before the beginning of the treatment with 18-beta glycyrrhetic acid. The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

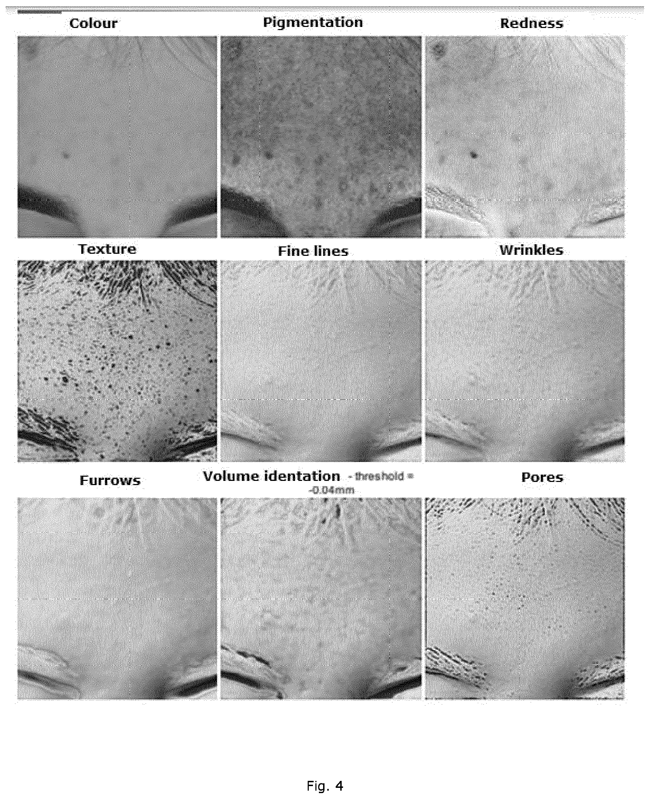

Figure 4 shows that after 21 days of application, the colour, redness and volumes parameters shown in Figure 3 have a positive variation. The colour parameter is attenuated with respect to the basal parameter; the redness parameter is reduced; the volumes parameter presents a reduction that can be evaluated as reduction.

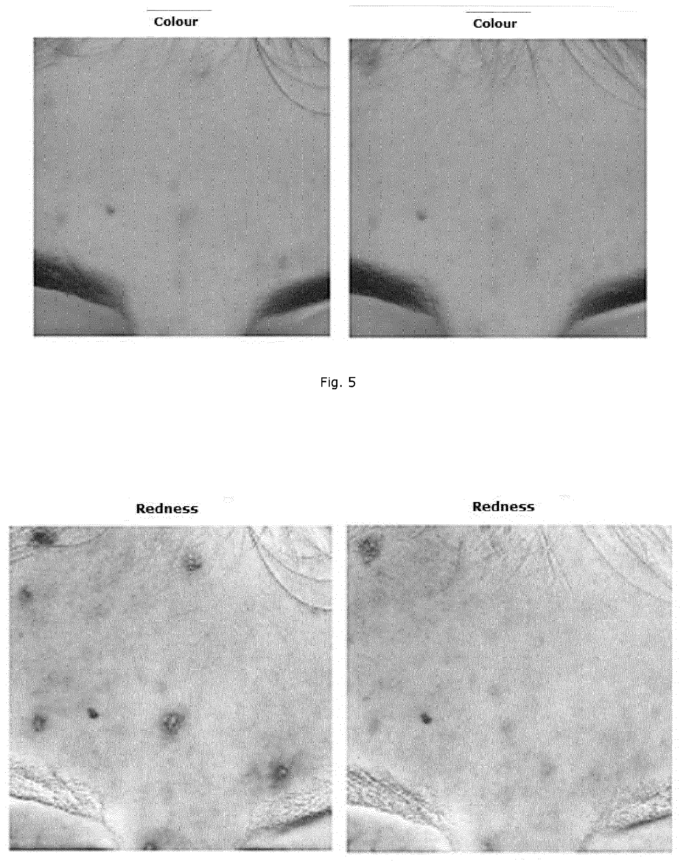

Figure 5 shows the reduction in the number of acne lesions at frontal-glabellar level of the patient under examination, treated with 18-beta glycyrrhetic acid.

Figure 6 shows that from analysis of the redness in the frontal-glabellar area, there is a marked reduction in the inflammatory lesions and in the erythematous areas both in number and dimension in the patient treated with 18-beta glycyrrhetic acid.

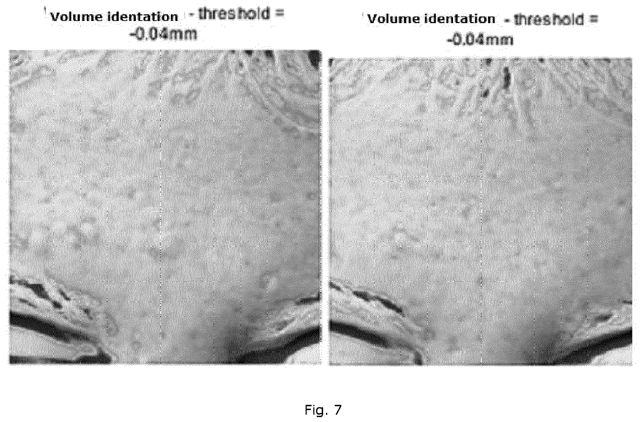

Figure 7 shows that after topical application of 18-beta glycyrrhetic acid there is a reduction in the volumes at frontal-glabellar level compatible with the reduction in the inflammatory lesions.

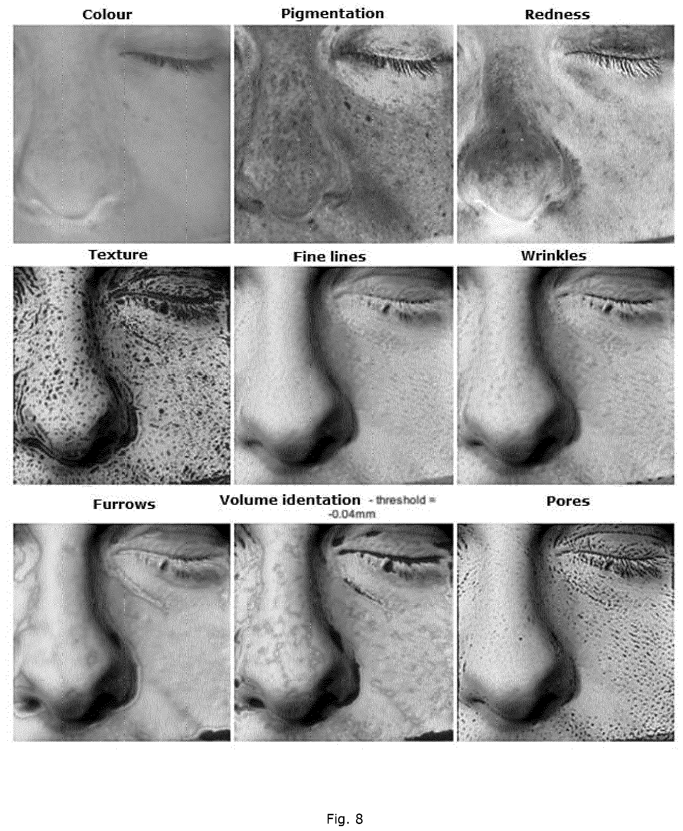

Figure 8 shows the 9 parameters examined during *in vivo* clinical evaluation on a human, at time T0, identified as baseline, namely before the beginning of the treatment with a composition comprising Vitamin C and nicotinamide, zinc, copper and intracellular glutathione (GSH). The skin colour corresponds to the one that can be seen by the operator in the macroscopic photo; the pigment highlights the chromophores and specifically the haemosiderin and the melanin at skin level; the redness highlights the surface capillarization; the texture highlights the skin irregularities as a variation of the skin trophism; the fine lines, wrinkles, volumes and furrows fall within the same colour spectrum identifying the skin depressions and the depth/height variations relative to the cutaneous plane; the pores represent the quantity and quality of the hair follicles.

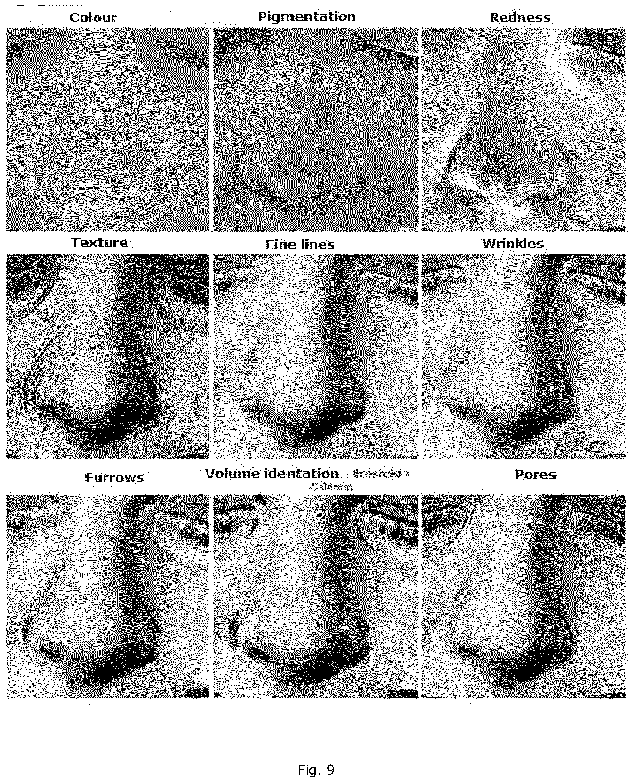

Figure 9 shows that after 21 days of application, 8 parameters examined show a positive variation. The colour parameter is attenuated with respect to the basal parameter; the pigmentation parameter is reduced, underlining the topical effect of reduction of the melanin and haemosiderin. A considerable improvement in the pores parameter is also noted, in terms of quantity and quality. No significant variation in the furrows parameter is noted.

### DETAILED DESCRIPTION

The present invention refers to a composition comprising nicotinamide, zinc, copper and intracellular glutathione (GSH).

Nicotinamide (the amide form of vitamin B3) has been used in dermatology for over 40 years for a wide range of conditions, including acne, rosacea, autoimmune bullous dermatitis and now the treatment and prevention of photoaging and photoimmunosuppression.

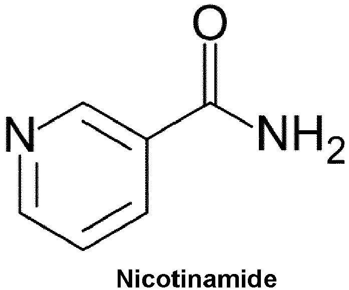

The broad clinical effects of nicotinamide can be explained by its role as a precursor of cell energy, modulator of the inflammatory cytokines and inhibitor of the nuclear enzyme poly (adenosine diphosphate-ribose) polymerase-1, which plays a significant role in DNA repair, maintenance of genomic stability and cell response to lesions, including inflammation and apoptosis. Its use in rosacea is due above all to its anti-inflammatory activities.

According to research conducted in recent years, zinc has the same properties as antibiotics like tetracycline and can efficiently combat acne bacteria, without the negative effects of the antibiotics, and improve the body's immunity. Furthermore, zinc is fundamental in the keratinization process, namely in correctly regulating keratin maturation, which is dysregulated during acne rosacea.

The Zn²⁺ ions reduce the production of reactive oxygen species (ROS) inhibiting the nicotinamide adenine dinucleotide phosphate (NADPH) oxidase. The list of the ROS, influenced by the production of Zn²⁺, comprises oxygenbased molecules comprising superoxide anion radical (^{.}O2-), hydrogen peroxide (H₂O₂) and hydroxyl radicals (^{.}OH), which are by-products of cell metabolism. However, at high concentrations, they can cause alterations in various molecules and damage the cell structures; the NADPH oxidase inhibitors like zinc could therefore have a protective influence taking account of the reduced formation of ROS.

Copper is an essential trace element for normal cell activity. It acts in fact as a cofactor for numerous enzymes, involved in various metabolic activities: in protection against oxidative stress, forming part of the structure of Superoxide dismutase and Ceruloplasmin; in the formation of connective tissue, participating in the activity of the enzyme lysyl-oxidase; in the synthesis of melanin, participating in the activity of the copper enzyme tyrosinase, necessary for formation of the pigment melanin (which plays a role in the pigmentation of hair, skin and eyes). The coenzymatic activity of the copper vis-à-vis enzymes like lysyl-oxidase guarantees correct synthesis and branching of the connective tissue, optimizing the formation of cross-linking between collagen fibres, thus structuring the matrix. Copper is able, via mechanisms that are still not fully clear, to control the activity of the cells responsible for the first response to potential pathogens, guaranteeing strengthening of the phagocytic activity of neutrophils and monocytes and providing effective protection against harmful agents of various types.

In the skin, copper performs a huge number of different activities, fundamental for carrying out physiological functions. It in fact stimulates proliferation of the dermal fibroblasts, upregulates the production of collagen (types I, II and V) and elastin fibres (elastin, fibrillins) by the fibroblasts, seemingly through the induction of TGF-β, stimulates HSp-47, essential for the formation of collagen fibrils, acts as a cofactor of LOX necessary for effective cross-linking of the extracellular matrix proteins, since the increase in cross-linking of the collagen and elastin matrixes depends on the copper dose.

However, in order to carry out its activities, copper must be correctly transported at intracellular level. Absorption of the copper depends on the concentrations of glutathione which, as seen previously, tend to decrease in inflammatory states. Therefore, to favour correct entry of the copper into the cell, it is essential to associate it with the glutathione, without which the copper would not be able to reach an efficient concentration in inflammatory states of acne rosacea. Furthermore, as said previously, the redox state is the basis of the inflammation, and the association between nicotinamide and glutathione favours the reduction in the production of ROS. Copper also intervenes in regulation of the redox state, since it is a cofactor of the superoxide dismutase, an antioxidant enzyme present in the skin, important for protection against free radicals, and above all, it inhibits cell oxidative effects like membrane damage and lipid peroxidation. Therefore, the rationale of associating the copper with the glutathione and nicotinamide is:
- in order to enter the cell, copper requires a sufficient quantity of glutathione, which is reduced in inflammatory states;
- at intracellular level, copper participates together with the glutathione in reducing the oxidative stress underlying acne rosacea;
- to achieve an adequate and lasting effect of the copper and glutathione at intracellular level, it is necessary to provide the glutathione with nicotinamide, which ensures regeneration of the glutathione in a reduced form, allowing it to act on the ROS, and to remain at concentrations that favour absorption of the copper;
- the zinc acts downstream, in turn favouring the formation of reduced nicotinamide, which will act as a substrate to form the reduced glutathione, which in turn acts in synergy with the copper to reduce the ROS and also to allow entry of the latter into the cell.

The intracellular glutathione (GSH) is the main scavenger protection mechanism against increase in oxidative stress. An altered GSH metabolism has been described in rosacea.

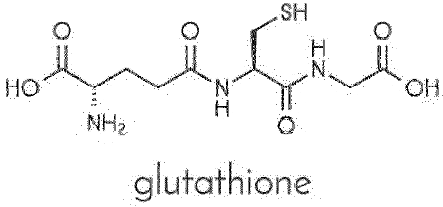

Chronic inflammation, like rosacea, is associated with oxidative stress. Oxidative stress occurs when the quantity of reactive oxygen species (ROS) exceeds the buffer capacity of the endogenous antioxidant defence system. Various studies have shown that oxidative stress is heavily involved in the pathogenesis of rosacea. In skin biopsies of patients with rosacea, the ROS levels are higher than in the healthy controls. The subjects with rosacea present increased ROS levels and a reduction in antioxidants like ascorbic acid also in the blood. UV radiation, a well-known factor triggering and aggravating rosacea, is a powerful inducer of the formation of ROS in the skin. The ROS are mediators of cytokine induction in human keratinocytes. Glutathione (GSH), a tripeptide formed of L-glutamate, cysteine and glycine, is the most abundant molecular low weight thiol in animal cells. GSH is present in millimolar concentrations in practically all normal cells. Therefore, GSH is the main intracellular antioxidant buffer against oxidative stress and exists mainly in the forms of reduced glutathione (GSH) and oxidised glutathione (GSSG). For these reasons, the therapeutic strategies aimed at increasing the intracellular levels of GSH could be, at least theoretically, an interesting tool for countering the pathogenetic role of oxidative stress in rosacea. However, GSH has a very low capacity for crossing the cell membrane. In fact, the intracellular levels of GSH are mainly the result of internal synthesis.

Nicotinamide plays a fundamental role in correct skin maturation, preventing damage induced by external factors. At the same time, nicotinamide intervenes in the redox balance of the cell, namely it balances the oxidation reactions and intracellular reduction. In fact, a correct level of nicotinamide is fundamental in continuation of the reduction and oxidation activities which occurs above all in inflammatory states. In particular, nicotinamide is able to favour regeneration of the reduced glutathione, one of the most important players in the inflammatory balance. Since glutathione is one of the main elements involved in cell inflammation regulation, and since acne rosacea has a significant inflammatory component, it has been seen that intracellular reduced glutathione tends to considerably decrease, with an imbalance tending towards inflammation. Maintaining high glutathione levels in inflammatory pathologies is fundamental. Therefore, the association of nicotinamide with glutathione allows adequate glutathione levels to be maintained also during the inflammatory stress of acne rosacea.

The ingredients described so far represent the main active ingredients in an embodiment of the present invention.

The active ingredient is defined as a substance that has a certain biological activity, therefore all substances having therapeutic effect like drugs or beneficial effect like vitamins and probiotics.

In one embodiment of the present invention, according to any one of the embodiments described here, the composition can further comprise one or more of the following substances: Vitamin C, Vitamin E, Papain, 18-β-Glycyrrhetic Acid, Hyaluronic acid.

Vitamin C (or ascorbic acid) is the vitamin "par excellence" as it takes part in numerous biological processes and physiological mechanisms that are "protective" for the organism. Normal skin contains high concentrations of vitamin C as it supports important and well-known functions, stimulating the synthesis of collagen and assisting the antioxidant protection against photodamage induced by UV rays. In the cosmetic field, Vitamin C is known above all for its powerful antioxidant activity. This is expressed through the scavenger effect, namely it scavenges the reactive oxygen species and participates in regeneration of α-tocopherol (Vitamin E), starting from its oxidised form. On the one hand, therefore, Vitamin C is able to provide direct anti-radical protection (it reduces the chemical reactivity and, consequently, the toxicity of the free radicals), and on the other, it is able to amplify the benefits ensured by other antioxidant substances (including vitamin E) and participates in the metabolic processes that lead to their physiological regeneration, making them once again useful in their important activity against the free radicals. Furthermore, Vitamin C is fundamental for maintaining the integrity of the collagen fibres of the blood vessels, fortifying them, and acting on one of the main causes of rosacea.

Vitamin E is an important liposoluble antioxidant and has been in use for over 50 years in dermatology. It is an important ingredient in many cosmetic products. It protects the skin from various harmful effects due to solar radiation, acting as a scavenger of the free radicals. Experimental studies suggest that Vitamin E has mitostatic and photoprotective properties. In one of the studies conducted on 98 patients affected by acne rosacea, control of the defective keratinisation was obtained with a compound of Vitamin E and Vitamin C, thus depriving the *Propionibacterium acnes* of the substrate necessary for its growth. Vitamin E prevents the lipid peroxidation induced by bacteria in the follicles and inside the sebaceous glands, thus preventing inflammation due to irritation of the peroxide.

Papain is a proteolytic enzyme present in the juice of the pawpaw (*Carica papaya*)*.* The results showed that papain performs its exfoliating effect by acting on the extracellular junctions that hold the keratinocytes together. This facilitates exfoliation of the surface layers of the skin, allowing greater penetration of the other active ingredients without increasing the inflammation.

One highly active vegetable ingredient is 18-β-glycyrrhetic acid (GA), the main metabolite of glycyrrhizin (GL), obtained from liquorice root. GA shows anti-inflammatory activity, suppressing the expression of pro-inflammatory genes, inhibiting the production of inflammatory cytokines and influencing the transformation of arachidonic acid into pro-inflammatory leukotrienes. Furthermore, GA has antioxidant properties and this activity is directed at inactivation of the free radicals and inhibition of the lipid oxidation. GA shows low toxicity against the normal human cell lines, the fibroblasts and the keratinocytes. Its substantial antimicrobial activity vis-à-vis some strains of bacteria indicates that GA could be a promising candidate for the treatment of diseases induced by skin microbes and disorders.

Hyaluronic acid is a simple linear polymer in which a simple disaccharide is repeated thousands of times, thus creating an enormous hydrophilic molecule that confers a large volume of hydration and contributes to the turgor and suppleness of healthy skin. An in-depth analysis of the literature has revealed that hyaluronic acid-based formulations (namely gels, creams, intradermal filler injections, dermal fillers, facial fillers, autologous fat gels, lotions, serums and prostheses, etc.) have considerable anti-ageing properties, with plumping, filling and rejuvenating effect on the face. Hyaluronic acid also performs an anti-inflammatory activity.

In a preferred embodiment of the present invention, the composition comprises: Hyaluronic Acid; Nicotinamide; Papain; Copper; Zinc; Glutathione; Vitamin C; 18-β-Glycyrrhetic Acid and Vitamin E.

The concentration range of the active ingredient of Hyaluronic acid present in the compositions described here can vary from 0.01% to 0.2%, and is preferably 0.1% (by weight with respect to the total weight of the composition).

With regard to the concentration of the active ingredient of Nicotinamide, the range described can vary from 0.01% to 0.5%, and is preferably 0.4% (by weight with respect to the total weight of the composition).

With reference to the concentration of Papain in the compositions described in the present invention, the range is between 0.01% and 0.2%, with preferred concentration of 0.1% (by weight with respect to the total weight of the composition).

Furthermore, the concentration of Copper present can be between 0.01% and 0.5%, and is preferably 0.2% (by weight with respect to the total weight of the composition).

The concentration of Zinc present in any one of the embodiments described in the present invention varies from 0.01% to 0.3%, and is preferably 0.1% (by weight with respect to the total weight of the composition).

As regards the concentration of the active ingredient of reduced Glutathione (GSH), the values can vary from 0.01% to 0.3%, and is preferably 0.1% (by weight with respect to the total weight of the composition).

The concentration values of Vitamin C can range from 0.01% to 0.3%, and is preferably 0.1% (by weight with respect to the total weight of the composition).

The concentration of 18-β-Glycyrrhetic Acid in the compositions described varies from 0.01% to 0.5%, with preferential value of 0.2% (by weight with respect to the total weight of the composition).

Lastly, the concentration value of Vitamin E ranges between 0.01% and 1%, and is preferably 0.7% (by weight with respect to the total weight of the composition).

Advantageously, in order to broaden the spectrum of protection provided by the present invention, Sun Protection Factors (SPF) can be further comprised in an embodiment.

The SPF indicates the degree of protection which a cosmetic product is able to guarantee against UV rays. It is an index expressed through a numerical value which is always specified on creams: the higher the number, the greater the protection offered against UV rays.

According to one of the embodiments of the present invention, the composition will comprise: Hyaluronic Acid (0.1%); Nicotinamide (0.4%); Papain (0.1%); Copper (0.2%) Zinc (0.1%); Glutathione (0.1%); Vitamin C (0.1%); 18-β-Glycyrrhetic Acid (0.2%); Vitamin E (0.7%); SPF filters; (percentages understood as weight of the component with respect to the total weight of the composition).

According to one of the embodiments of the present invention, the composition is used topically, and will therefore be preferably in the form of: cream, ointment, pomade, lotion, toner, gel, paste, foam, hydrophobic gel, hydrophilic gel, emulsion, suspension or liquid.

According to the type of formulation, the composition can further comprise one or more excipients and/or carriers. For lipophilic ointments, the excipients can be chosen from: solid, semi-solid and/or liquid paraffins, vegetable oils, waxes, liquid silicones, which generally have occlusive or protective properties. It is also possible to add surfactants like sorbitan esters, lanolin alcohols, fatty alcohols, fatty acid sulphates, esters of fatty acids and/or polysorbates to obtain an ointment that emulsifies in water.

With regard instead to the composition of the present invention in the form of hydrophilic ointment, polyethylene glycol mixtures are appropriate excipients to be added.

With reference to a possible embodiment in which the composition described by the present invention is in the form of cream, since cream is a multi-phase preparation consisting of a lipophilic phase and a hydrophilic phase, the addition of emulsifiers is fundamental. In the case of hydrophobic creams, the emulsifiers that can be added are wool alcohols, esters of sorbitan and/or monoglycerides, whereas for hydrophilic creams, the emulsifiers will be sodium soaps, polysorbates and/or sulphates of fatty alcohols.

With regard to compositions formulated as hydrophilic gels, the addition of hydrophilic macromolecules, such as sodium alginate, agar-agar, gums, gelatin, starch, derivatives of cellulose and/or carboxy-vinyl-polymers is essential.

In the case of hydrophobic gel, excipients that can be included are: micronized silica, hydrogenated castor oil, stearyl-ammonium hectorite, zinc stearates, calcium stearates and/or aluminium stearates.

Other excipients can be hydrating substances like collagen, elastin, various protein hydrolysates, nucleic acid hydrolysates, natural moisturizing factor; or absorption promoters, like DMSO, fatty acids, urea-azone and/or menthol.

A further subject of the present invention is the development of a cosmetic product comprising or consisting of: Nicotinamide, Copper, Zinc, Glutathione and/or Vitamin C and/or 18-β-Glycyrrhetic Acid and/or Vitamin E and/or Hyaluronic Acid and/or Papain.

The cosmetic product in question, according to the present invention, can be conceived in the form of: serum, face mask, make-up remover wipe, cleansing wipe, moisturizing cream, lifting cream, moisturizing gel, make-up remover, exfoliant, toner, micellar water, make-up product.

All the embodiments described so far can be prepared by means of any one of the known methods used in the cosmetic sector.

The composition described in the present description can be used as a medicament.

In particular, this medicament can be used in the treatment of acne rosacea, capillary fragility, telangiectasia and facial pustulosis.

Lastly, the invention claims the cosmetic (non-therapeutic) use of the composition according to any one of the embodiments previously described, in order to prevent, reduce and/or eliminate skin imperfections such as, for example, flushing and couperouge, in particular on the face.

The use of said composition entails application in the morning, after thoroughly cleansing and drying the area of interest, and if necessary in the evening, but in any case at least once a day, gently massaging until complete absorption.

### EXAMPLES

Example 1 - Hyaluronic Acid (0.1%); Nicotinamide (0.4%); Papain (0.1%); Copper (0.2%) Zinc (0.1%); Glutathione (0.1%); Vitamin C (0.1%); 18-β-Glycyrrhetic Acid (0.2%); Vitamin E (0.7%); with SPF filters.
**Appearance:** emulsion
**Colour:** green
**Flavour:** ND
**Smell:** characteristic
**pH:** 5.5-6.5
**Density:** 0.95- 0.99 g/ml
**Refraction index:** ND
**Flash point:** non-flammable
**Solubility in water:** non-soluble

### Materials and Methods

In order to demonstrate the synergic activity of the ingredients of the composition of the invention, 18 subjects were recruited at the dermatological clinic of Policlinico di Roma Tor Vergata. The subjects recruited were divided into 6 arms (3 subjects per arm) in order to evaluate the effect of the complete composition and the single active ingredients after 21 days of topical application, twice a day. In each arm, based on a particular topical formulation, 9 parameters were evaluated: colour, pigmentation, fine lines, wrinkles, pores, redness, texture, volumes and furrows. The parameters were evaluated using an *in vivo* skin imaging method by means of Miravex (Limited features Antera 3D, Dublin).

### Interim results

**Hyaluronic Acid (0.1%); Nicotinamide (0.4%); Papain (0.1%); Copper (0.2%); Zinc (0.1%); Glutathione (0.1%); Vitamin C (0.1%); 18-β-Glycyrrhetic Acid (0.2%); Vitamin E (0.7%); with spf filters:** the present composition showed an improvement in all 9 parameters examined (skin colour, pigmentation, fine lines, wrinkles, pores, redness, texture, volumes), further showing that the furrows parameter is improved only in the above-mentioned composition, underlining the synergy of all the ingredients, without being improved by the individual ingredients.

**base cream/carrier + Vitamin C (0.1%):** the active ingredient showed improvement in the redness, colour and volume parameters without significant alteration of the pores parameter.

**base cream/carrier + 18-β-glycyrrhetic acid (0.2%):** the active ingredient showed improvement in the redness, colour and volume parameters without significant alteration in the other parameters.

**Table 1:**

| Composition | **skin colour** | **pigmentation** | **fine lines** | **wrinkles** | **pores** | **redness** | **texture** | **volumes** | **furrows** |
|---|---|---|---|---|---|---|---|---|---|
| Complete formulation | X | X | X | X | X | X | X | X | X |
| Vitamin E alone | | | | | | | | | |
| Vitamin C and hyaluronic acid | X | X | X | X | X | X | X | X | |
| Hyaluronic acid alone | | | | | | | | | |
| Papain alone | | | | | | | | | |
| 18-β-glycyrrhetic acid alone | X | | | | | X | | X | |

## Claims

1. Composition comprising nicotinamide, zinc, copper and intracellular glutathione (GSH).

2. The composition according to claim 1, further comprising one or more of the following substances Vitamin E, Vitamin C, Hyaluronic acid, Papain, 18-β-glycyrrhetic acid.

3. The composition according to any one of claims 1 to 2, comprising: Hyaluronic acid; Nicotinamide; Papain; Copper; Zinc; Glutathione; C vitamin; 18-β-glycyrrhetic acid and Vitamin E.

4. The composition according to any one of claims 1 to 3, wherein the hyaluronic acid is present in a range between 0.01% and 0.2%, preferably 0.1%; and/or
Nicotinamide is present in a range between 0.01% and 0.5%, preferably 0.4%; and/or
Papain is present in a range between 0.01% and 0.2%, preferably 0.1%; and/or
wherein Copper is present in a range between 0.01% and 0.5%, preferably 0.2%; and/or
Zinc is present in a range between 0.01% and 0.3%, preferably 0.1%; and/or
wherein Glutathione is present in a range between 0.01% and 0.3%, preferably 0.1%; and/or
wherein Vitamin C is present in a range between 0.01% and 0.3%, preferably 0.1%; and/or
wherein 18-β-glycyrrhetic acid is present in a range between 0.01% and 0.5%, preferably 0.2%; and/or
wherein Vitamin E is present in a range between 0.01% and 1%, preferably 0.7%.

5. The composition according to any one of claims 1 to 4, further comprising sun protection factors (SPF).

6. The composition according to any one of claims 1 to 5, for topical use.

7. The composition according to claim 6, in the form of a cream, ointment, ointment, lotion, tonic, gel, paste, foam, hydrophobic gel, hydrophilic gel, emulsion, suspension or liquid.

8. A cosmetic product comprising or consisting of a composition according to any one of claims 1 to 7, wherein said product is in the form of a serum, face mask, make-up remover wipe, cleansing wipe, moisturizing cream, lifting cream, moisturizing gel, make-up remover , exfoliating, tonic, micellar water, make-up product.

9. Cosmetic use of the composition, according to any one of claims 1 to 8, to prevent, reduce and/or eliminate skin imperfections, in particular flushing and/or couperouge localized on the face.

10. Composition according to any one of claims 1 to 7, as a medicament.

11. Composition according to any one of claims 1 to 7 for use in the treatment of acne rosacea, flushing, couperouge, capillary fragility, facial pustulosis and/or telangiectasias.
